# EUROPÄISCHE PATENTSCHRIFT

(11) **EP 2 316 931 B1**
(45) Veröffentlichungstag und Bekanntmachung des Hinweises auf die Patenterteilung: **28.12.2016**
(21) Anmeldenummer: 10153604.3
(22) Anmeldetag: 15.02.2010
(51) Int. Cl.: C12N 9/64, C12N 9/74, G01N 33/86

(54) **Polymergekoppelte Peptidasen**
Polymer-coupled peptidases
Peptidases couplées par polymère

(30) Priorität: 30.10.2009 EP 09013711
(43) Veröffentlichungstag der Anmeldung: 04.05.2011
(73) Patentinhaber: Senova Gesellschaft für Biowissenschaft und Technik mbH, 99427 Weimar (DE)
(72) Erfinder: Kolde, Hans-Jürgen, 85521 Ottobrunn (DE); Lange, Ute, 07768 Kahla (DE); Bucha, Elke, 99094 Erfurt (DE)
(74) Vertreter: dompatent von Kreisler Selting Werner - Partnerschaft von Patent- und Rechtsanwälten mbB

(56) Entgegenhaltungen:
- WO-A2-2006/134174
- WO-A2-2009/042962
- DE-A1- 19 904 674
- SEN ET AL.: "Effect of glycoPEGylation on factor VIIa binding and internalization" HAEMOPHILIA, Bd. 16, 21. Oktober 2009 (2009-10-21), Seiten 339-348, XP002573945

## Beschreibung

Peptidasen (auch als Proteinasen oder Proteasen bezeichnet) sind Enzyme, die die Spaltung von Peptidbindungen in Peptiden oder Proteinen katalysieren. In Form der Endopeptidasen bevorzugen sie als Reaktionspartner Peptidbindungen innerhalb des Zielmoleküls, an denen zumeist bestimmte Aminosäuren beteiligt sind. Andere Peptidasen spalten vom N- oder C-Terminus des Proteins einzelne oder mehrere Aminosäuren ab. Dabei sind die verschiedenen Endopeptidasen in Abhängigkeit von ihrer Tertiärstruktur oft in der Lage, mit verschiedenen, spezifische Peptidbindungen enthaltenden physiologischen oder unphysiologischen Reaktionspartnern unterschiedlicher Molekülgröße zu reagieren.

Peptidasen haben wichtige Funktionen im Organismus und sind an vielfältigen Vorgängen wie z.B. bei der Eiweißverdauung, der Immunantwort (Komplementsystem), der Blutgerinnung und der Fibrinolyse in ihren physiologischen aber auch pathologischen Abläufen beteiligt.

In manchen Fällen bilden die aktivierten Endopeptidasen dazu auch noch makromolekulare Komplexe mit Kofaktoren, so z.B. der aktivierte Gerinnungsfaktor Xa mit Faktor Va im Prothrombinasekomplex.

Für bestimmte diagnostische aber auch therapeutische Verfahren ist dieses Spektrum an Reaktionspartnern für einen gezielten, spezifischen Einsatz derartiger Enzyme jedoch von Nachteil.

Der Prozess der Blutgerinnung ist ein sehr komplexer mehrstufiger Vorgang, in dem inaktive Enzymvorstufen durch Enzymwirkungen unter Mitwirkung von Kofaktoren zu aktiven Gerinnungsenzymen aktiviert werden.

Zur Ausbalancierung des Gleichgewichtes zwischen der Gerinnungsbereitschaft des Blutes und der Gewährleistung seiner Fließeigenschaften im Kreislauf existiert außerdem ein vernetztes System von hemmend oder verstärkend wirkenden Interaktionen mit weiteren Molekülen, das durch zahlreiche positive und negative Rückkopplungsmechanismen gesteuert wird.

Für die Behandlung von Störungen des Blutgerinnungssystems wurden und werden neue Arzneimittelklassen entwickelt, die ganz gezielt nur ein einziges Enzym der Gerinnungskaskade hemmen, wie z.B. den aktivierten Faktor Xa (Wirkstoffklasse der direkt wirkenden Faktor Xa-Hemmstoffe z.B. Rivaroxaban®, Apixaban®, Betrixaban®, Otamixaban®, Edoxaban®, Eribaxaban, YM150, LY-517717, PRT054021 u.a.) oder das Thrombin (Wirkstoffklasse der direkt wirkenden Thrombinhemmstoffe, z.B. Dabigatran®, Argatroban, Bivalirudin, MCC-977, AZD0837, NU172, Flovagatran u.a.).

Zur Gewährleistung einer effektiven (Thromboseverhinderung) und sicheren Therapie (Blutungsverhinderung) mit solchen Arzneimitteln ist sowohl die Verfügbarkeit von entsprechenden exakten Nachweismethoden des Wirkstoffes in Körperflüssigkeiten als auch die Möglichkeit, diese Arzneimittel neutralisieren oder entfernen zu können, bedeutsam.

Um Arzneimittel, die spezifisch Enzyme des Hämostasesystems hemmen, gezielt messen oder sie in vitro oder in vivo neutralisieren zu können, wäre es von großem Vorteil das Spektrum der Reaktionsmöglichkeiten der jeweiligen Enzyme modifizieren bzw. begrenzen zu können.

Entsprechend sind für die Antidot-Entwicklung gegen Faktor Xa- und Thrombinhemmstoffe Modifikationen der Molekülstruktur von Thrombin und Faktor Xa bekannt geworden, durch die diese Peptidasen ihre Funktions- und Interaktionsfähigkeit im Gerinnungssystem verlieren, aber noch mit den spezifischen lnhibitorwirkstoffen interagieren. Derart modifizierte Peptidasen sollen zur Neutralisation des Wirkstoffes eingesetzt werden. Als Neutralisation wird dabei eine Bindung des Wirkstoffmoleküls an die Peptidase bezeichnet, die dazu führt, dass das Wirkstoffmolekül seine Funktion als Inhibitor des Hämostasesystems nicht mehr ausüben kann. Erreicht wird dies durch das Entfernen spezieller Aminosäuren und / oder durch deren Austausch. Hierfür sind aufwändige Verfahren u.a. offenbart in US 6 060 300 (Thrombin muteins as antidotes for thrombin inhibitors) und in WO 2009/041962 (Antidotes for Factor Xa inhibitors and methods of using the same). US 2009/0098119A1 beschreibt darüber hinaus, dass die an sich kurze Halbwertszeit der Mutanten von Faktor Xa durch eine Umsetzung mit Polyethylenglycol verlängert werden kann, aber nicht, dass sich durch diese Modifikation andere Eigenschaften des mutierten FaktorXa- Moleküls ändern.

Aufgabe der vorliegenden Erfindung war es, Verfahren zu Detektion oder quantitativen Bestimmung eines Inhibitors einer Peptidase des Hämostasesystems in einer Probe bereitzustellen.
Weiterhin war es Aufgabe der vorliegenden Erfindung, in-vitro Verfahren zur Neutralisation der inhibitorischen Aktivität eines Inhibitors einer Peptidase des Hämostasesystems bereitzustellen.

Offenbart wird auf einfache Weise die Spezifität von Peptidasen des Hämostasesystems, wie z. B. Peptidasen des Gerinnungssystems, zu verändern, insbesondere um die derart modifizierten Peptidasen zum Nachweis oder zur Neutralisation von Arzneimitteln einsetzen zu können, die als Hemmstoffe auf Peptidasen des Gerinnungsystems wirken. Offenbart wird die Bereitstellung von modifizierten Peptidasen, die mit den nachzuweisenden bzw. zu neutralisierenden Inhibitoren und Substraten reagieren können, jedoch ihre Reaktionsfähigkeit im Gerinnungssystem im Wesentlichen verlieren.

Es wurde überraschend festgestellt, dass Peptidasen des Hämostasesystems oder Gerinnungssystems, wie Faktor Xa oder Thrombin, nach Kopplung an ein oder mehrere Polymere, insbesondere Polyalkylenglycole und Copolymere, die Alkylenglycol-Einheiten umfassen, ihre Reaktionsfähigkeit im Gerinnungssystem verlieren, aber gleichzeitig noch mit Inhibitoren und Substraten von niederer Molekülgröße reagieren können. Die derart modifizierten Peptidasen des Hämostasesystems werden im Folgenden zur Vereinfachung auch als "polymergekoppelte Peptidasen" bezeichnet.

Die Peptidase, die hier mit einem oder mehreren Polymeren gekoppelt wird, ist eine Peptidase des Hämostasesystems, insbesondere des Hämostasesystems eines Säugers, wie dem Menschen. Sie kann auf unterschiedliche Art und Weise gewonnen werden, und schließt sowohl rekombinante Peptidasen als auch aus Organismen, insbesondere aus Blut gewonnene Peptidasen sowie deren Fragmente oder Mutanten ein. Mutanten der Peptidasen unterscheiden sich von den entsprechenden nativen Peptidasen dadurch, dass ein oder mehr, vorzugsweise 1 bis 10 Aminosäurereste deletiert oder insertiert oder durch jeweils einen Aminosäurerest ausgetauscht sind. Sie können durch bekannte Verfahren, wie beispielsweise synthetische Verfahren oder ortsgerichtete Mutagenese bereit gestellt werden. Idealerweise zeigen die Fragmente und Mutanten der hier genannten Peptidasen mindestens 80 %, stärker bevorzugt 90 % der biologischen Aktivität und insbesondere dieselbe biologische Aktivität wie die entsprechende native Peptidase, wobei die Aktivität mit auf dem Fachgebiet bekannten Verfahren bestimmt werden kann. Das heißt, das entsprechende Fragment bzw. die Mutante, wenn nicht an ein Polymer gekoppelt, hat bevorzugt die gleiche physiologische Wirkung und interagiert mit den gleichen Substanzen mit der gleichen biologischen Wirkung wie die entsprechende native Peptidase.

Bevorzugt handelt es sich bei der Peptidase des Hämostasesystems um einen Gerinnungsfaktor in seiner aktivierten Form. Offenbart wird ein Gerinnungsfaktor ausgewählt aus Faktor IIa (Thrombin), VIIa, IXa, Xa und XIa. Besonders bevorzugt sind Thrombin und Faktor Xa. Ebenfalls bevorzugt ist der Einsatz einer nativen Form der Proteinasen zur Kopplung an ein oder mehrere Polymere.

Die hier an derartige Peptidasen zu koppelnden Polymere sind chemische Verbindungen aus Molekülketten und/oder verzweigten Molekülen, die bevorzugt aus gleichen oder gleichartigen Einheiten bestehen. Dazu gehören synthetische Polymere wie z.B. Polyalkylenglycole oder auch Biopolymere, wie z.B. Moleküle mit sich wiederholenden, Aminosäuresequenzen oder Kohlenhydrateinheiten, die ggf. durch Substitution derivatisiert werden können. Beispiele sind Dextran oder Hydroxyethylstärke. In der Arzneimittelindustrie werden einige dieser Polymere an Wirkstoffe gekoppelt, um auf diese Weise deren Halbwertszeit im Organismus zu verlängern. Solche Verfahren sind z.B. die PEGylierung, HESylierung und PASylierung.

Das Polymer, das hier an eine Peptidase des Hämostasesystems gekoppelt wird, hat üblicherweise eine mittlere molekulare Masse (Gewichtsmittel) von mindestens 1.000 Da, bevorzugt mindestens 2000 Da und insbesondere mindestens 5.000 Da. Darüber hinaus beträgt die molekulare Masse üblicherweise höchstens 60.000, bevorzugt höchstens 40.000 Da und insbesondere höchstens 20.000 Da.

Das Polymer kann verzweigt oder linear sein, lineare Polymere sind bevorzugt.

Offenbart wird für das Polymer der polymergekoppelten Peptidase auch ein Polyalkylenglycol oder ein Copolymer, das Alkylenglycol-Einheiten umfasst. Der Alkylenanteil der Alkylenglycol-Einheiten des Polyalkylenglycols oder des Copolymers umfasst bevorzugt 1 bis 6, besonders bevorzugt 2 oder 3 C-Atome. Copolymere enthalten bevorzugt mindesten 50 mol %, besonders bevorzugt mindestens 70 mol % und insbesondere mindestens 90 mol %, bezogen auf die Gesamtmenge der Wiederholungseinheiten im Copolymer, an Wiederholungseinheiten der Formel -Alk-O-, wobei "Alk" für eine Alkylengruppe wie vorstehend definiert steht.

Besonders bevorzugt handelt es sich bei dem Polymer um ein Polyethylenglycol (auch als PEG bezeichnet) oder ein Copolymer, das Ethylenglycol-Einheiten umfasst. Copolymere enthalten bevorzugt mindesten 50 mol %, besonders bevorzugt mindestens 70 mol % und insbesondere mindestens 90 mol %, bezogen auf die Gesamtmenge der Wiederholungseinheiten, an Wiederholungseinheiten der Formel -CH₂-CH₂-O-.

In einer besonders bevorzugten Ausführungsform handelt es sich bei der Peptidase um den Gerinnungsfaktor Xa und bei dem Polymer um Polyethylenglycol mit einer mittleren molekularen Masse (Gewichtsmittel) von üblicherweise mindestens 1.000 Da, bevorzugt mindestens 2000 Da und insbesondere mindestens 5.000 Da. Darüber hinaus beträgt die mittlere molekulare Masse üblicherweise höchstens 60.000, bevorzugt höchstens 40.000 Da und insbesondere höchstens 20.000 Da. In einer weiteren besonders bevorzugten Ausführungsform handelt es sich bei der Peptidase um den Gerinnungsfaktor Thrombin und bei dem Polymer um Polyethylenglycol mit einer molekularen Masse (Gewichtsmittel) von üblicherweise mindestens 1.000 Da, bevorzugt mindestens 2000 Da und insbesondere mindestens 5.000 Da. Darüber hinaus beträgt die mittlere molekulare Masse üblicherweise höchstens 60.000, bevorzugt höchstens 40.000 Da und insbesondere höchstens 20.000 Da.

Die Kopplung des Polymers an die Peptidase erfolgt vorzugsweise durch eine kovalente Bindung, die mit chemischen Standardverfahren erzeugt werden kann. In der Regel wird das Polymer dazu an einem Terminus aktiviert, um mit einer funktionellen Gruppe der Peptidase reagieren zu können. Im Fall eines Polyalkylenglycols können dabei bekannte reaktive Derivate wie beispielsweise Succinimidylsuccinat, Succinimidylpropionat, Nitrophenylcarbonat, Tresylat, Epoxide, Aldehyde, Isocyanate, Maleimide und dergleichen verwendet werden (Veronese FM, Pasut G: PEGylation, successful approach to drug delivery. DDT 2005; 10:1451-1458). Ein Peptidasemolekül kann an eine oder mehrere Polymermoleküle, beispielsweise 1 bis 10 Polymermoleküle gekoppelt werden. Das molare Verhältnis Peptidase/Polymer wird durch die eingesetzten Mengen bei der Kopplungsreaktion gesteuert.

Es wurde festgestellt, dass durch die Kopplung der Peptidase mit dem Polymer die Spezifität, d.h. insbesondere die Substratspezifität der Peptidase so modifiziert wird, dass die Peptidase ihre Reaktionsfähigkeit im Hämostasesystem verliert oder die Reaktionsfähigkeit signifikant eingeschränkt ist. Insbesondere verliert sie ihre Reaktionsfähigkeit mit makromolekularen physiologischen Rezeptoren, makromolekularen Substraten, makromolekularen Kofaktoren und makromolekularen Inhibitoren. Bei solchen makromolekularen physiologischen Rezeptoren, makromolekularen Substraten, makromolekularen Kofaktoren und makromolekularen Inhibitoren handelt es sich häufig um Proteine und Polypeptide. In diesem Zusammenhang bedeutet der Verlust bzw. die signifikante Einschränkung der Reaktionsfähigkeit im Hämostasesystem beispielsweise, dass die Peptidase nicht mehr bzw. nur noch in minimalem Umfang in der Lage ist, ihre physiologische Funktion zu erfüllen und/oder die genannten makromolekularen Substrate umzusetzen. Dies kann z.B. für polymergekoppelte Gerinnungsfaktoren dadurch gezeigt werden, dass diese in einem kommerziell erhältlichen Plasma mit Mangel an dem entsprechenden Gerinnungsfaktor (z.B. abnormal plasma, American Diagnostica Inc.) die durch diesen Faktorenmangel verlängerte Prothrombinzeit des Mangelplasmas nicht beeinflussen, während die Zugabe einer unmodifizierten Peptidase des Gerinnungssystems zu einer Verkürzung bis Normalisierung der Prothrombinzeit des Plasmas führt. Auch mit Hilfe von auf dem Fachgebiet bekannten Gerinnungsassays lässt sich feststellen, dass die polymergekoppelte Peptidasen keine Verkürzung der Gerinnungszeit als Zeichen einer Gerinnungswirkung in Plasmaproben hervorrufen.

Beispielsweise kann durch die Polymerkopplung die Reaktionsfähigkeit einer Peptidase mit makromolekularen Substanzen des Hämostasesystems mit einer molekularen Masse von 30.000 Da und höher unterdrückt bzw. signifikant eingeschränkt werden.

Auf diese Weise geht durch die Polymerkopplung nicht nur die Fähigkeit der Peptidase verloren, mit Reaktionspartnern der Gerinnungskaskade aktivierend zu reagieren, sondern sie können ebenfalls nicht mehr von physiologischen Inhibitoren wie z.B. Antithrombin mit einer molekularen Masse von 58.000 Da (insbesondere für PEG-Xa, PEG-IIa) oder tissue factor pathway inhibitor mit einer molekularen Masse von 39.000 Da (insbesondere für Faktor Xa) gehemmt werden.

Dagegen wird durch die Kopplung mit dem Polymer die Fähigkeit der Peptidase nicht beeinträchtigt, mit niedermolekularen Substraten oder niedermolekularen Inhibitoren der Peptidase des Hämostasesystems zu reagieren. Die Reaktion mit einem Inhibitor der Peptidase führt in diesem Zusammenhang zu einer Hemmung der Aktivität der polymergekoppelten Peptidase. Je nach Konzentrationsverhältnis zwischen Peptidase und Inhibitor kann es sich dabei um eine teilweise oder vollständige Hemmung handeln. Dabei bezeichnet der Begriff der Aktivität der polymergekoppelten Peptidase insbesondere ihre Fähigkeit, Substrate enzymatisch umzusetzen. Da, wie vorstehend beschrieben, die Reaktionsfähigkeit der Peptidase mit makromolekularen Substraten durch die Kopplung mit dem Polymer nicht mehr gegeben ist, handelt es sich in der Regel um die Fähigkeit, niedermolekulare Substrate enzymatisch umzusetzen. Offenbart wird ein Substrat, das eine molekulare Masse von mindestens 100 Da und höchstens 10.000 Da aufweist. Ebenfalls offenbart beträgt die molekulare Masse des Substrats höchstens 7.500 Da, insbesondere höchstens 5.000 Da oder höchstens 2.500 Da.

Inhibitoren von Peptidasen des Hämostasesystems, die im Rahmen der Erfindung auch als Inhibitoren der polymergekoppelten Peptidasen Wirkung zeigen sind üblicherweise direkt interagierende bzw. direkt bindende Inhibitoren oder direkt bindende Gerinnungshemmstoffe, insbesondere solche, die als Arzneistoffe zu diesem Zweck eingesetzt werden.

In der Regel handelt es sich bei den Inhibitoren des Hämostasesystems, die mit den polymergekoppelten Peptidasen reagieren bzw. von ihnen neutralisiert werden, um niedermolekulare Inhibitoren. Offenbart wird ein Inhibitor, der eine molekulare Masse von mindestens 100 Da und höchstens 10.000 Da aufweist. Ebenfalls offenbart beträgt die molekulare Masse des Inhibitors höchstens 7.500 Da, insbesondere höchstens 5.000 Da oder höchstens 2.500 Da. Beispiele solcher Inhibitoren sind Wirkstoffe, ausgewählt aus der Gruppe der Wirkstoffe der Arzneimittel zur direkten Hemmung von Gerinnungsfaktor Xa wie Rivaroxaban, Apixaban, Betrixaban, Otamixaban, Edoxaban, Raxazaban, Eribaxaban, YM150, LY-517717 oder PRT054021 oder zur direkten Hemmung von Thrombin, wie Dabigatran, Argatroban, Flovagatran, AZD0837, MCC-977, NU172 oder Bivalirudin. Als Wirkstoffe zur direkten Hemmung oder direkte Inhibitoren werden dabei solche Stoffe bezeichnet, die ihre hemmende Aktivität über eine direkte Wechselwirkung mit dem Gerinnungsfaktor, d.h. typischerweise eine Bindung an den entsprechenden Gerinnungsfaktor, ausüben.

In einer besonders bevorzugten Ausführungsform zur Bereitstellung einer polymergekoppelten Peptidase erfolgt eine Kopplung der Peptidase an PEG nach den an sich bekannten Verfahren. Hierbei können reaktive PEG-Derivate wie beispielsweise Succinimidylsuccinat, Succinimidylpropionat, Nitrophenylcarbonat, Tresylat, Epoxide, Aldehyde, Isocyanate, Maleimide und dergleichen verwendet werden (Veronese FM, Pasut G: PEGylation, successful approach to drug delivery. DDT 2005; 10:1451-1458).

Die Zahl der zu koppelnden Ketten sowie ihre Länge kann beliebig gestaltet und für die jeweilige Enzymstruktur adaptiert werden, wobei bevorzugt mittlere molekulare Massen der PEG-Ketten wie vorstehend allgemein angegeben sind. Um die Reaktionsfähigkeit von Peptidasen mit Bestandteilen des Gerinnungssystems vollständig auszuschalten, werden vorzugsweise zur Kopplung PEG-Moleküle von mindestens 5 000 Da verwendet.

Auf diese Weise kann z.B. Faktor Xa derartig an PEG gekoppelt werden, dass der entstandene PEG-Xa nicht mehr in der Lage ist, mit dem Prothrombinasekomplex (Kofaktor Va, Phospholipid) zu interagieren und das Proenzym Prothrombin zum aktiven Thrombin, dem für die Gerinnungsauslösung wesentlichen Enzym am Ende der Gerinnungskaskade, zu spalten. PEG-Xa verliert dabei ebenfalls seine Fähigkeit, mit den physiologischen makromolekularen Inhibitoren von Faktor Xa wie Antithrombin oder TFPI (tissue factor pathway inhibitor) zu reagieren.

Dagegen bleibt nach der PEG-Kopplung an Faktor Xa überraschenderweise die ursprüngliche Reaktionsfähigkeit von Faktor Xa mit den u.a. als Arzneimittelwirkstoffen verwendeten niedermolekularen Hemmstoffen erhalten, ebenso wie die Reaktionsfähigkeit mit niedermolekularen Peptidsubstraten.
Eine Ausführungsform der Erfindnung, bei der die hier beschriebenen polymergekoppelten Peptidasen zum Einsatz kommen, ist ein Verfahren zur Detektion oder quantitativen Bestimmung eines Inhibitors einer Peptidase des Hämostasesystems in einer Probe, wobei die Peptidase des Hämostasesystems ein Gerinnungsfaktor ist, ausgewählt aus der Gruppe bestehend aus Faktor IIa (Thrombin), VIIa, IXa, Xa, XIa sowie Fragmenten und Mutanten der genannten Peptidasen, die mindestens 80% der Fähigkeit der genannten Peptidasen ihre Substrate des Gerinnungssystems enzymatisch umzusetzen zeigen und die Probe ausgewählt ist aus Blut, Plasma, Serum, Liquor, Urin, Schweiß oder einer anderen Flüssigkeit, umfassend das in Kontakt bringen des Inhibitors in der Probe mit der an ein Polymer gekoppelten Peptidase des Hämostasesystems, und das Polymer ein Polyalkylenglycol oder ein Copolymer ist, das Alkylenglycol-Einheiten umfasst, und wobei die Peptidase des Hämostasesystems durch die Polymerkopplung ihre Reaktionsfähigkeit im Gerinnungssystem verliert, aber gleichzeitig noch Inhibitoren des Gerinnungssystems mit einer molekularen Masse zwischen 100 und 2.500 Da neutralisieren und Substrate des Gerinnungssystems mit einer molekularen Masse zwischen 100 und 2.500 Da enzymatisch umsetzen kann, und entweder
(i) das Messen der Aktivität der polymergekoppelten Peptidase nach deren in Kontakt bringen mit dem Inhibitor in der Probe, und den Vergleich der Aktivität der gekoppelten Peptidase nach dem in Kontakt bringen mit der Probe mit einem oder mehreren Referenzwerten, um eine eventuelle Hemmung der Aktivität festzustellen; oder
(ii) das Messen der Gerinnungszeit der Probe nach dem in Kontakt bringen mit der gekoppelten Peptidase, und den Vergleich der Gerinnungszeit mit einem oder mehreren Referenzwerten, um eine eventuelle Änderung der Gerinnungszeit festzustellen. Ausgelöst wird eine derartige Änderung durch die Reaktion des Inhibitors mit der gekoppelten Peptidase, was zu einer Hemmung der Aktivität des Inhibitors führt.

Dieses Verfahren eignet sich insbesondere zur Detektion oder quantitativen Bestimmung eines niedermolekularen Inhibitors. Offenbart wird wiederum ein Inhibitor, der eine molekulare Masse von mindestens 100 Da und höchstens 10.000 Da aufweist. Ebenso offenbart beträgt die molekulare Masse des Inhibitors höchstens 7.500 Da, insbesondere höchstens 5.000 Da oder höchstens 2.500 Da. Beispiele solcher Inhibitoren sind wie vorstehend genannt. Es sollte für den Fachmann klar sein, dass üblicherweise die im Verfahren eingesetzte polymergekoppelte Peptidase an den zu detektierenden oder quantitativ zu bestimmenden Inhibitor angepasst werden sollte, d.h. es sollte eine Peptidase ausgewählt werden, die grundsätzlich zu einer Interaktion mit dem Inhibitor in der Lage ist. Zur Detektion oder quantitativen Bestimmung eines niedermolekularen Inhibitors im Rahmen der Erfindung sollte vorzugsweise eine polymergekoppelte Peptidase eingesetzt werden, auf die der Inhibitor als direkter Inhibitor wirkt.

Insbesondere kann das Verfahren zur Detektion oder quantitativen Bestimmung die folgenden einleitenden Schritte umfassen:
(a) Bereit stellen mindestens einer Probe, in der ein Inhibitor vorliegen soll; und
(b) Kontaktieren mindestens einer Probe aus (a) mit einer polymergekoppelten Peptidase wie hier beschrieben.

Dabei können in Schritt (a) gewonnene Proben entsprechend Verfahren (ii) zur Detektion oder quantitativen Bestimmung des Inhibitors nicht nur als Messprobe in Schritt (b), sondern auch als Vergleichsproben herangezogen werden, z.B. zur Messung einer Gerinnungszeit ohne Zusatz der PEGgekoppelten Peptidase.

Das in Kontakt bringen der gekoppelten Peptidase mit dem Inhibitor im erfindungsgemäßen Verfahren umfasst bevorzugt eine Inkubation der Probe mit der gekoppelten Peptidase, insbesondere in Schritt (b), während derer eine Interaktion eines gegebenenfalls in der Probe vorhandenen Inhibitors mit der gekoppelten Peptidase stattfinden kann.

Offenbart wird, dass es sich bei der Probe um eine Probe handelt, die eine Flüssigkeit umfasst bzw. eine Flüssigkeit ist. Häufig handelt es sich um eine biologische Probe, beispielsweise eine Körperflüssigkeit, wie Blut, Plasma, Serum, Liquor, Urin oder Schweiß, bevorzugt Blut oder Plasma. Bei der Probe kann es sich beispielsweise um eine Probe für ein Mess- oder Diagnoseverfahren handeln.

Nach Option (i) des oben genannten Verfahrens kann beispielsweise ein Referenzwert für den Vergleich erzeugt werden, indem die Aktivität der polymergekoppelten Peptidase ohne vorherigen Kontakt mit einem Inhibitor gemessen wird. Eine Verringerung der Aktivität der gekoppelten Peptidase, nachdem sie mit einem Inhibitor in der Probe reagiert hat, im Vergleich zu einem solchen Referenzwert, erlaubt z.B. den Schluss, dass der Inhibitor in der Probe vorhanden war. Nach Option (i) können aber auch mehrere Referenzwerte für den Vergleich erzeugt werden, indem die Aktivität der gekoppelten Peptidase nach Kontakt mit unterschiedlichen bekannten Konzentrationen eines Inhibitors gemessen wird. So erlaubt beispielsweise der Vergleich mit den Referenzwerten eine quantitative Bestimmung des Inhibitors.

Die Messung der Aktivität der polymergekoppelten Peptidase, sowohl vor in Kontakt bringen mit der Probe zum Erzeugen eines oder mehrerer Referenzwerte als auch nach dem in Kontakt bringen mit der Probe zum Erzeugen des Messwerts, der Aufschluss über das Vorliegen bzw. die Konzentration des Inhibitors in der Probe gibt, kann nach herkömmlichen Verfahren erfolgen. Dabei wird insbesondere die Fähigkeit der polymergekoppelten Peptidase bestimmt, Substrate enzymatisch umzusetzen. Da, wie vorstehend beschrieben, die Reaktionsfähigkeit der Peptidase mit makromolekularen Substraten durch die Kopplung mit dem Polymer nicht mehr gegeben ist, handelt es sich in der Regel um die Fähigkeit, niedermolekulare Substrate enzymatisch umzusetzen. Offenbart ist dabei ein Substrat, das eine molekulare Masse von mindestens 100 Da und höchstens 10.000 Da aufweist. Ebenfalls offenbart beträgt die molekulare Masse des Substrats höchstens 7.500 Da, insbesondere höchstens 5.000 Da oder höchstens 2.500 Da. Beispielsweise können bekannte Substrate eingesetzt werden, von denen die Peptidase eine signalgebende Gruppe abspalten kann, wie z.B. ein chromogenes Substrat. Als Beispiele geeigneter kommerziell erhältlicher chromogener Substrate für Peptidasen kann für Faktor Xa N-α-Z-D-Arg-Gly-Arg-pNA (z.B S-2765™, Chromogenix Instrumentation Laboratory, Milano, Italy) genannt werden, und kann für Thrombin H-D-Phe-Pip-Arg-pNA (S-2238™, Chromogenix Instrumentation Laboratory, Milano, Italy), genannt werden, als Alternativ können z.B. auch fluorigene, luminogene oder elektrochemische Substrate zusammen mit der entsprechenden Detektionstechnik verwendet werden.

Nach Option (ii) kann der Inhibitor in der Probe über die Messung der Gerinnungszeit bestimmt werden. Hierfür stehen unterschiedliche Möglichkeiten zur Verfügung. Für eine Detektion genügt es in der Regel festzustellen, dass sich die Gerinnungszeit einer Probe, die mit der polymergekoppelten Peptidase in Kontakt gebracht wurde, gegenüber der Gerinnungszeit einer entsprechenden Probe ohne Kontakt mit der gekoppelten Peptidase (Referenzwert) durch eine vollständige oder anteilige Neutralisation des Inhibitors verkürzt.

Für eine quantitative Bestimmung kann ein entsprechender Vergleich von zwei Proben bzw. Gruppen von Proben herangezogen werden, von denen in einer der Inhibitor durch Zugabe der gekoppelten Peptidase neutralisiert wurde. Die beobachtete Änderung der Gerinnungszeit in den neutralisierten gegenüber den nicht neutralisierten Proben kann anschließend z.B. mit Hilfe einer Eichkurve in Relation gesetzt werden mit Änderungen, die durch bekannte Konzentrationen an Inhibitoren Hervorgerufen werden. In einer spezifischen Ausführungsform der Option (ii) kann durch die Zugabe einer definierten Menge der gekoppelten Peptidase der in der Probe enthaltenen Inhibitor anteilig neutralisiert werden. Wird dieser Probe nun ein Gerinnungsaktivator, wie z.B. Thromboplastin, partielles Thromboplastin, gerinnungsaktivierendes Schlangengift oder eine daraus isolierte gerinnungsaktivierende Protease oder ein aktivierter Gerinnungsfaktor, der in der Aktivierungskaskade vor dem gehemmten Enzym steht, zugesetzt, wird die Gerinnungszeit durch die Restkonzentration des Inhibitors im Vergleich zu einer entsprechenden Probe ohne Inhibitor verlängert. Vergleicht man diesen Wert mit der Gerinnungszeit, die im gleichen Probenmaterial ohne Zusatz der gekoppelten Peptidase sowie unter Zusatz bekannter Konzentrationen eines geeigneten Kalibrators gemessen wurden, kann bei Verwendung einer geeigneten Kalibration die Konzentration des Inhibitors unter Ausschluss des Einflusses der Probenzusammensetzung bestimmt werden.

Ist das Probenmaterial auf Grund seiner Zusammensetzung nicht in der Lage zu gerinnen, kann für die Gerinnungsmessung der Option (ii) z.B. Normalplasma als Quelle für andere Gerinnungsfaktoren oder insbesondere Fibrinogen zugesetzt werden, wodurch sich die Spezifität des Verfahrens noch verbessert.

Bei dem Verfahren der ersten Ausführungsform kann es sich z.B. um ein *in-vitro-*diagnostisches Verfahren, insbesondere zum Nachweis oder zur quantitativen Messung eines Inhibitors einer Peptidase des Hämostasesystems in einer biologischen Probe, wie Blut, Plasma, Serum, Liquor, Urin oder Schweiß handeln. Das Verfahren kann aber z.B. auch in Screening-Verfahren bei der Wirkstoffsuche eingesetzt werden.

Die Probe kann, z.B. im Rahmen eines in-vitro diagnostischen Verfahrens, insbesondere von einem Säuger, wie z.B. einem Menschen stammen.

In einer besonders bevorzugten Ausführungsform zur Detektion oder quantitativen Bestimmung eines Inhibitors einer Peptidase des Hämostasesystems wird ein spezifischer Nachweis von gerinnungshemmenden Arzneimitteln, deren Wirkstoff ein direkter FaktorXa-Inhibitor ist, in Körperflüssigkeiten wie Blut, Plasma, Serum, Liquor, Urin, Schweiß oder in anderen Flüssigkeiten ermöglicht. Auf Grund der Spezifität des polymergekoppelten Faktor Xa, insbesondere von PEG gekoppeltem Faktor Xa (nachstehend auch "Polymer-Xa" bzw. "PEG-Xa") für direkte Faktor Xa Inhibitoren erlaubt dieses erfindungsgemäße Verfahren exakte Aussagen zur Wirkstoffkonzentration, ohne dass das Ergebnis durch den Einfluss des aktuellen Funktionszustandes des Gerinnungssystems verfälscht wird. Ebenfalls ohne Einfluss sind indirekte, z.B. über Antithrombin wirkende FaktorXa-Inhibitoren, wie unfraktionierte oder niedermolekuare Heparine, Orgaran oder Pentasaccharide wie Fondaparinux. Dieser Aspekt ist in so fern ein Vorteil, da oft Patienten von Heparinen auf direkte parenterale oder orale Antikoagulanzien umgestellt werden und daher in einer Übergangszeit beide Wirkstoffklassen im Blut vertreten sind. Diese werden daher in bisherigen Bestimmungsmethoden in mehr oder weniger starkem Umfang mit erfasst.

Zur Durchführung des bevorzugten erfindungsgemäßen Verfahrens wird eine Probe, z.B. Plasma, die einen direkt wirkenden Faktor-Xa-Inhibitor enthält, mit einer definierten Menge an polymergekoppeltem Xa, insbesondere PEG-Xa, versetzt und über einen geeigneten Zeitraum inkubiert. Das Polymer-Xa bzw. PEG-Xa bindet den Inhibitor und wird dabei anteilig inaktiviert. Zugesetzt wird nun ein Substrat, von dem das nicht inaktivierte Polymer-Xa bzw. PEG-Xa eine signalgebende Gruppe abspalten kann, wie z.B. ein chromogenes Substrat.

Die mit der Konzentration des inhibitors invers korrelierende Restaktivität an Polymer-Xa bzw. PEG-Xa spaltet das chromogene Substrat. Die daraus resultierende Farbentwicklung wird mit einem geeigneten Verfahren gemessen. Durch Verwendung einer entsprechenden Kalibration kann die Konzentration des Inhibitors in der Probe exakt und unbeeinflusst von der Probenzusammensetzung, insbesondere auch in Gegenwart von indirekt wirkenden Hemmstoffen, wie Heparinen, gemessen werden. Neben insbesondere niedermolekularen chromogenen Substraten können z.B. auch niedermolekulare fluorigene, luminogene oder elektrochemische Substrate zusammen mit der entsprechenden Detektionstechnik verwendet werden.

Die Grundsätzlichkeit des Verfahrens zur Modifikation der Spezifität von Peptidasen des Hämostasesystems durch Kopplung an Polymere wie Polyethylenglycol kann auch am Beispiel des Thrombins gezeigt werden (nachfolgend auch Polymer-Thrombin bzw. PEG-Thrombin). In seiner polymergekoppelten Form, insbesondere durch Kopplung mit PEG, verliert Thrombin seine Fähigkeit mit Bestandteilen des Gerinnungssystems zu interagieren und damit seine Funktion im Gerinnungssystem. Es ist nicht mehr in der Lage Fibrinogen zu Fibrin zu spalten und damit in einer Plasma- oder Blutprobe die Gerinnung auszulösen. Polymer-Thrombin, insbesondere PEG-Thrombin, kann weder mit weiteren Bestandteilen des plasmatischen und zellulären Gerinnungssystems noch mit physiologischen Inhibitoren wie Antithrombin, Heparinkofaktor II oder α2-Makroglobulin reagieren.

Dagegen ist beim Polymer-Thrombin, insbesondere PEG-Thrombin, die Reaktionsfähigkeit mit direkt wirkenden niedermolekularen Thrombininhibitoren mit einem bevorzugten Molekulargewicht von mindestens 100 Da und höchstens 7.500 Da erhalten, ebenso wie die Reaktionsfähigkeit mit niedermolekularen Peptidsubstraten mit entsprechenden Molekulargewichten.

Demzufolge kann entsprechend des bevorzugten Verfahrens zur Messung von Faktor Xa-Hemmstoffen z.B. auch ein Verfahren zur Messung direkt wirkender niedermolekularer Thrombinhemmstoffe durchgeführt werden. Polymer-Thrombin, insbesondere PEG-Thrombin, bindet hierbei den in der Probe enthaltenen Inhibitor und wird dadurch anteilig inaktiviert. Seine Restaktivität, die mit der Konzentration des Inhibitors in der Probe invers korreliert, wird mit geeigneten niedermolekularen Substraten, z.B. durch die Bestimmung der Farbintensität nach Reaktion mit einem chromogenen Substrat gemessen. Durch Verwendung einer entsprechenden Kalibration kann die Konzentration des Inhibitors bestimmt werden.

Auf Grund der Eigenschaften der polymergekoppelten, insbesondere PEG-gekoppelten Peptidasen sind auch bevorzugte Verfahren zur Messung der Konzentration von direkt wirkenden Hemmstoffen von Faktor Xa und Thrombin etabllerbar, die wie vorstehend beschrieben, auf einer Gerinnungszeitmessung basieren. Hierfür wird durch die Zugabe einer definierten Menge des entsprechenden PEG-Peptidase der in der Probe enthaltene Hemmstoff anteilig neutralisiert. Wird in dieser Probe nun durch Zusatz geeigneter Gerinnungsaktivatoren, wie z.B. Thromboplastin, partielles Thromboplastin, gerinnungsaktivierendes Schlangengift oder eine daraus isolierte gerinnungsaktivierende Protease oder durch Zusatz eines aktivierten Gerinnungsfaktors der in der Aktivierungskaskade vor der gehemmten Peptidase steht zugesetzt, wird die Gerinnungszeit durch die Restkonzentration des Wirkstoffes verlängert. Vergleicht man diesen Wert mit der Gerinnungszeit, die im gleichen Probenmaterial ohne Zusatz des PEG-Enzyms sowie unter Zusatz bekannter Konzentrationen eines geeigneten Kalibrators gemessen wurde, kann die Konzentration des Gerinnungshemmstoffes unter Ausschluss des Einflusses der Probenzusammensetzung bestimmt werden. Ist das Probenmaterial auf Grund seiner Zusammensetzung nicht in der Lage zu gerinnen, kann Normalplasma als Quelle für andere Gerinnungsfaktoren und insbesondere Fibrinogen zugesetzt werden, wodurch sich die Spezifität des Verfahrens noch verbessert.

Verfahren nach diesen Prinzipien sind prinzipiell neben Faktor Xa und Thrombin auch für andere Peptidasen des Hämostasesystems bzw. ihre Inhibitoren möglich, wie z.B. für die Faktoren IXa, Xla und VIIa.

Weiterhin offenbart sind auch Vorrichtungen, Arzneimittel und Verfahren zur Neutralisation von Inhibitoren von Peptidasen des Hämostasesystems, wobei der Begriff der Neutralisation so zu verstehen ist, dass der Inhibitor durch eine Reaktion mit der erfindungsgemäßen gekoppelten Peptidase gebunden wird und so keine weitere inhibitorische Aktivität in einer Probe oder in einem Organismus mehr zeigen kann.

Umfasst ist von der vorliegenden Erfindung ein *in vitro* Verfahren zur Neutralisation der inhibitorischen Aktivität eines Inhibitors einer Peptidase des Hämostasesystems mit einer molekularen Masse zwischen 100 und 2.500 Da, wobei die Peptidase ausgewählt ist aus der Gruppe bestehend aus Faktor IIa (Thrombin), VIIa, IXa, Xa, XIa sowie Fragmenten und Mutanten der genannten Peptidasen, die mindestens 80% der Fähigkeit der genannten Peptidasen zeigen, ihre Substrate des Gerinnungssystems enzymatisch umzusetzen, umfassend das in Kontakt bringen einer Probe ausgewählt aus Blut, Plasma, Serum, Liquor, Urin, Schweiß oder einer anderen Flüssigkeit, in der der Inhibitor enthalten ist, mit einer polymergekoppelten Peptidase des Hämostasesystems, und es sich bei dem Polymer um Polyalkylenglycol oder ein Copolymer handelt, das Alkylenglycol-Einheiten umfasst, und wobei die Peptidase durch die Polymerkopplung ihre Reaktionsfähigkeit im Gerinnungssystem verliert, aber gleichzeitig noch Inhibitoren des Gerinnungssystems mit einer molekularen Masse zwischen 100 und 2.500 Da neutralisieren und Substrate des Gerinnungssystems mit einer molekularen Masse zwischen 100 und 2.500 Da enzymatisch umsetzen kann, und das Binden des Inhibitors durch Reaktion mit der polymergekoppelten Peptidase.

Als Probe offenbart ist eine Probe, die eine Flüssigkeit umfasst bzw. eine Flüssigkeit ist. Häufig handelt es sich um eine biologische Probe, beispielsweise eine Körperflüssigkeit, wie Blut, Plasma, Serum, Liquor, Urin oder Schweiß, bevorzugt Blut oder Plasma. In einer bevorzugten Ausführungsform wird dieses Verfahren im Rahmen einer *in vitro* Diagnose eingesetzt, bei der eine Probe einem Säuger, insbesondere einem Menschen, entnommen aber nicht zurück geführt wird.

In einer Ausführungsform wird dabei die Probe aus dem Blut eines Patienten gewonnen, der mit einem Arzneimittel behandelt wird oder wurde, das einen Inhibitor einer Peptidase des Hämostasesystems als Wirkstoff enthält. Nach dem in Kontakt bringen der Probe mit der gekoppelten Peptidase wird der mit dem Arzneimittel verabreichte Inhibitor durch die Peptidase gebunden und damit neutralisiert. Im Rahmen des Verfahrens können daher zusätzlich nach der Neutralisation des Inhibitors Tests zur Diagnose von Störungen im Hämostasesystem oder zur Aktivität von Substanzen, die in das Hämostasesystem eingreifen auf vorteilhafte Weise an der Probe durchgeführt werden.

Bei Patienten die mit direkt wirkenden gerinnungshemmenden Inhibitoren behandelt werden, bewirkt die Anwesenheit dieser Inhibitoren im Blut bzw. Plasma, dass bestimmte in vitro Labormethoden für die Diagnostik des Funktionszustandes des Hämostasesystems oder zur Aktivitätsbestimmung von Substanzen, die in das Hämostasesystem eingreifen, erheblich gestört und damit nicht mehr aussagefähig sind. Dies betrifft insbesondere Methoden, die auf der Messung der Gerinnungszeit vom Moment der Zugabe spezieller Aktivatoren bis zum Eintritt des Gerinnungsereignisses beruhen (z.B. aPTT, PT, TT, Fibrinogenbestimmung). Unter dem Einfluss der Arzneiwirkstoffe wird die Gerinnungszeit in diesen Tests aber bereits durch diese Wirkstoffe artifiziell deutlich verzögert und lässt keine diagnostischen Rückschlüsse auf den Zustand des Hämostasesystems mehr zu. Insbesondere kann kein Mangel an Gerinnungsfaktoren mehr diagnostiziert werden, was in verschiedenen klinischen Situationen von Nachteil ist. Durch die Neutralisation der Inhibitoren können diese Tests ohne Beeinflussung durch den Inhibitor durchgeführt werden und behalten ihre volle diagnostische Aussagekraft.

Auch für diese Ausführungsform sind Inhibitoren offenbart , die eine molekulare Masse von mindestens 100 Da und höchstens 10.000 Da aufweisen.Ebenfalls offenbart beträgt die molekulare Masse des Inhibitors höchstens 7.500 Da, insbesondere höchstens 5.000 Da oder höchstens 2.500 Da. Beispiele solcher Inhibitoren sind wie vorstehend genannt. Zur Neutralisation des Inhibitors im Rahmen der Erfindung sollte vorteilhafterweise eine polymergekoppelte Peptidase eingesetzt werden, auf die der Inhibitor als direkter Inhibitor wirkt.

Offenbart ist auch eine Vorrichtung, die eine erfindungsgemäße polymergekoppelte Peptidase des Hämostasesystems enthält, zum Entfernen eines Inhibitors einer Peptidase des Blutgerinnungssystems aus einer Probe oder aus dem Blutkreislauf eines Patienten. Dabei kann die polymergekoppelte Peptidase beispielsweise auf einem Trägermaterial immobilisiert sein. Bezug genommen wir in diesem Zusammenhang beispielhaft auf die WO 98/46648, die Verfahren und Interaktionssysteme offenbart, mit deren Hilfe die polymergekoppelten Peptidasen, insbesondere Polyalkylenglycol oder PEG-gekoppelte Peptidasen, auf einem Trägermaterial immobilisiert werden können. Die Immobilisierung der polymergekoppelten Peptidase kann z.B. an partikulären Strukturen, Kapillaren, Netzstrukturen oder Behälterwänden erfolgen. Die immobilisierte gekoppelte Peptidase kann insbesondere in einem Behälter vorliegen, wie einer Kartusche, der einen Einlass und einen Auslass für Flüssigkeiten aufweist und von einer Flüssigkeit durchströmt werden kann.

Besonders geeignet sind solche Formen der Vorrichtung, die sich extrakorporal in den Blutkreislauf eines Patienten schalten lassen. Hierfür kann z.B. Blut mittels eines Schlauchsystems mit oder ohne Pumpe aus dem Organismus extrakorporal durch eine Kartusche geleitet werden, die mit einem geeigneten Material wie z.B. Kapillaren, Partikel o.ä. gefüllt ist, an dessen Oberfläche die polymergekoppelte Peptidase immobilisiert ist. Strömt das Blut oder auch Plasma, wenn zuvor eine Zellseparation vorgenommen wurde, an der oberflächenfixierten polymergekoppelten Peptidase vorbei, bindet diese spezifisch den enthaltenen Inhibitor, der auf diese Weise aus der Zirkulation entfernt wird, was zu einer Absenkung der Blutkonzentration in nicht toxische Bereiche führt.

Offenbart ist auch eine polymergekoppelte Peptidase des Hämostasesystems zur Verwendung bei der Wiederherstellung der Gerinnungsfähigkeit des Blutes, zur Erhöhung der Gerinnungsneigung des Blutes und/oder zur Beschleunigung der Blutgerinnung.

Eine derartige Verwendung kann insbesondere angezeigt sein bei Patienten, die einer Behandlung mit einem niedermolekularen Gerinnungshemmstoff unterzogen wurden bzw. werden. Bevorzugt ist dabei wiederum ein Inhibitor, der eine molekulare Masse von mindestens 100 Da und höchstens 10.000 Da aufweist. Bevorzugt beträgt die molekulare Masse des Inhibitors höchstens 7.500 Da, insbesondere höchstens 5.000 Da oder höchstens 2.500 Da. Beispiele solcher Inhibitoren sind Wirkstoffe, ausgewählt aus der Gruppe der Wirkstoffe der Arzneimittel zur direkten Hemmung von Gerinnungsfaktor Xa wie z.B. Rivaroxaban, Apixaban, Betrixaban, Otamixaban, Edoxaban, Eribaxaban, YM150, LY-517717 oder PRT054021 oder zur direkten Hemmung von Thrombin, wie z.B. Dabigatran, Argatroban, Flovagatran, AZD0837, MCC-977, NU172 oder Bivalirudin.

Solche polymergekoppelten Peptidasen zur Neutralisierung von Inhibitoren, insbesondere direkt wirkenden niedermolekularen Gerinnungshemmstoffen können z.B. bei Patienten Anwendung finden, z.B. wenn die Blutkonzentration dieser Wirkstoffe in Bereichen liegt, die zu gefährlichen Blutungen führen können, z.B. um damit eine Operation zu ermöglichen. Hierfür kann die entsprechende polymergekoppelte Peptidase direkt in den Kreislauf des Patienten appliziert werden. Sie wird im Blut spezifisch mit dem Gerinnungshemmstoff reagieren und dessen gerinnungshemmende Wirkung damit antagonisieren, ohne selbst eine Eigenwirkung auszuüben. Im Gegensatz zu Mutanten von Gerinnungsfaktoren ist hier keine Immunisierung zu befürchten, da es sich vorzugsweise um die natürliche Form des Proteins handeln kann. Das Beispiel der Hemmkörperhämophilie zeigt, dass selbst minimal Änderungen der Struktur durch Punktmutationen bereits eine klinisch bedeutsame Antikörperbildung induzieren kann. Die polymergekoppelte Peptidase kann aber auch in immobilisierter Form mit dem Blut eines Patienten in Kontakt gebracht werden, hierbei wird auf die vorstehend offenbarte Vorrichtung verwiesen.

Offenbart ist auch ein Testkit, umfassend als ein erstes Reagenz eine polymergekoppelten Peptidase des Hämostasesystems und als ein zweites Reagenz ein Substrat der Peptidase, das in den vorstehend beschriebenen Diagnoseverfahren zum Einsatz kommen kann.

Insbesondere können im Kit bekannte Substrate eingesetzt werden, von denen die polymergekoppelte Peptidase eine signalgebende Gruppe abspalten kann, wie z.B. ein chromogenes Substrat. Alternativ können z.B. auch fluorigene, luminogene oder elektrochemische Substrate verwendet werden. Es dürfte verständlich sein, dass sich für diese Zwecke aufgrund der Modifikation der Peptidase insbesondere niedermolekulare Substrate eignen, bevorzugt solche, die eine molekulare Masse von mindestens 100 Da und höchstens 10.000 Da aufweisen. Besonders bevorzugt beträgt die molekulare Masse höchstens 7.500 Da, insbesondere höchstens 5.000 Da oder höchstens 2.500 Da.

Die nachfolgenden Beispiele sollen die Erfindung näher erläutern, ohne sie darauf zu beschränken.

### Herstellung eines PEG-Faktor Xa Konjugates

150*µ*g boviner Faktor Xa (EC 3.4.21.6, American Diagnostica Inc.) gelöst in 300*µ*l 0,05M Phosphatpuffer, pH 8,0 wurden zu 20mg Methoxy-Polyethylenglycol (PEG)-20.000 Succinimidylproprionat gegeben. Der Ansatz wurde 1h im Kühlschrank (+2°C -+8°C) geschüttelt. Nach 1 h wurden dem Ansatz weitere 10mg Methoxy-PEG-20,000 succinimidylproprionat zugesetzt und das Gemisch wurde eine weitere Stunde bei +2°C -+8°C geschüttelt.

Das PEG 20kD-Factor Xa Konjugat wurde durch Größenausschlusschromatographie an einer Hi Load Superdex 200pg 16/60 Säule mit 0,02M Tris/HCl, 0,1 M NaCl, pH 7.4 bei einer Flussrate von 1 ml/min isoliert. Das PEG-Factor Xa Konjugat wird als symmetrischer Peak bei einem Elutionsvolumen von 40ml zuerst eluiert (Detektion der UV Absorption bei 220nm und 280nm). Der Polymerüberschuss und die Reaktionsprodukte werden später eluiert und wurden auf diese Weise vom konjugierten Protein getrennt.

Die Protein-haltigen Fraktionen mit Faktor Xa Aktivität (bestimmt über die Spaltung eines chromogenen Substrates für Faktor Xa) wurden in Fraktionssammlerröhrchen gesammelt, in denen PEG 8000 als Stabilisator vorgelegt wurde. Die Fraktionen mit dem höchsten Gehalt an Faktor Xa wurden gepoolt und in Aliquots bei -80°C aufgewahrt.

### BEISPIEL 2

### Enzymatische Eigenschaften von PEG 20kD-Faktor Xa

### a) Spaltung von chromogenem Substrat durch PEG 20kD-Faktor Xa

Die Spaltung des chromogenen Substrates N-α-Z-D-Arg-Gly-Arg-pNA (Haemochrom Diagnostica GmbH) wurde über die Bestimmung der Michaelis-Menthen Konstante Kₘ des Substrates charakterisiert. Als Kₘ für PEG 20kD-Faktor Xa wurde 0,102mM ermittelt, die Konstante ist damit identisch mit Kₘ für den unmodifizierten Faktor Xa, die 0,105mM betrug.

### b) Hemmung der amidolytischen Aktivität von PEG 20kD-Faktor Xa durch den niedermolekularen direkten Inhibitor Pefabloc Xa

Um die Hemmung der amidolytischen Aktivität von PEG 20kD-Faktor Xa durch den niedermolekularen Inhibitor Pefabloc Xa (Loxo GmbH) zu ermitteln, wurde ein chromogener Assay und die Auswertung nach Lineweaver-Burk eingesetzt. Die Inhibitorkonstante Kⱼ für Pefabloc Xa gegenüber PEG 20kD-Faktor Xa betrug 0.7 ± 0.13*µ*M. Sie ist vergleichbar mit der gegenüber dem unmodifizierten Enzym ermittelten Inhibitor konstante Kᵢ von 1.1 ± 0.07*µ*M.

### c) Einfluss von Antithrombin und Heparin/Antithrombin auf die amidolytische Aktivität von PEG 20kD-Faktor Xa

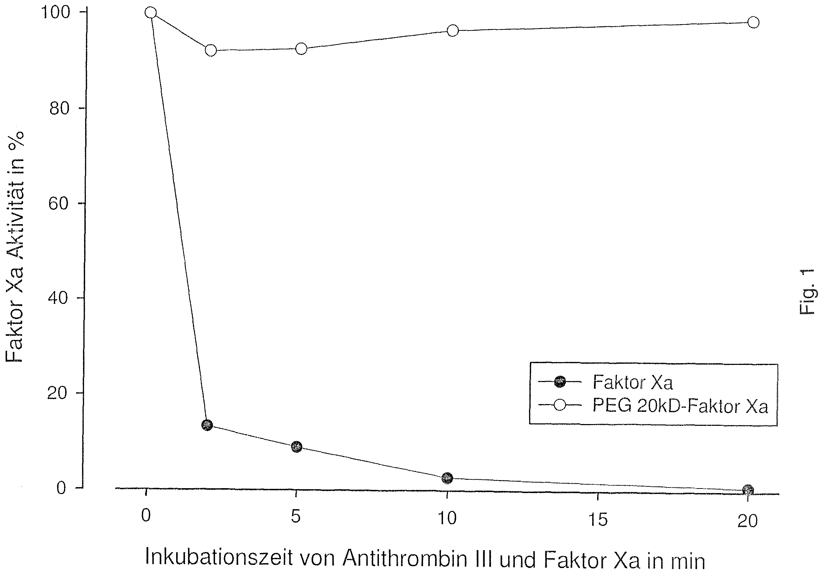

25*µ*l Faktor Xa (American Diagnostica Inc., 1*µ*g/ml) bzw. PEG 20kD-Faktor Xa (1*µ*g Protein/ml) wurden mit 25*µ*l Antithrombin III (50 units/ml, Sigma Chemical Co. A-7388) 2-20min lang inkubiert, die amidolytische Aktivität wurde nach Zugabe von chromogenem Substrat bestimmt. Während die Aktivität von Faktor Xa in Abhängigkeit von der Inkubationszeit um 99% gehemmt wurde, hatte Antithrombin III keinen inhibitorischen Einfluss auf die PEG 20kD-Faktor Xa-Aktivität (Abbildung 1).

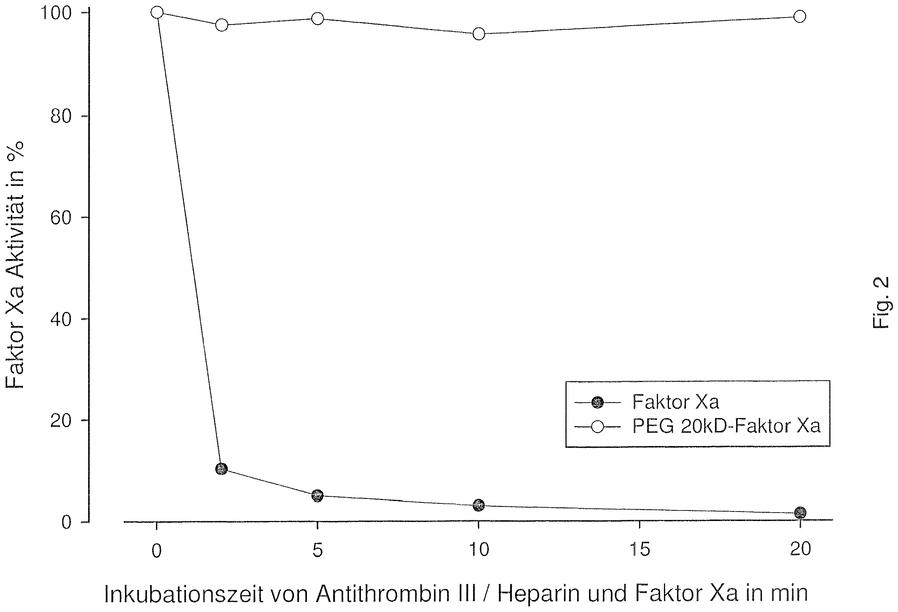

25*µ*l Faktor Xa (American Diagnostica Inc., 1*µ*g/ml) bzw. PEG 20kD-Faktor Xa (1*µ*g Protein/ml) wurden mit 25*µ*l eines Gemischs aus Antithrombin III (10 units/ml, Sigma Chemical Co. A-7388) und Heparin (2 anti-FXa units/ml, Low molecular weight heparin, 2nd International Standard, NIBSC) 2-20min lang inkubiert, die amidolytische Aktivität wurde nach Zugabe von chromogenem Substrat bestimmt. Während die Aktivität von Faktor Xa in Abhängigkeit von der Inkubationszeit um 99% gehemmt wurde, hatte der Antithrombin III / Heparin Komplex keinen inhibitorischen Einfluss auf die PEG 20kD-Faktor Xa-Aktivität (Abbildung 2).

### d) Aktivität von PEG 20kD-Faktor Xa im plasmatischen Gerinnungssystem

Die Gerinnungsaktivität von nicht modifiziertem Faktor Xa (American Diagnostica Inc.) wurde bestimmt, indem 50*µ*l Plasma mit 50*µ*l Faktor Xa (Proteingehalt zwischen 0,015*µ*g/ml und 2*µ*g/ml) für 1 min bei 37°C inkubiert wurden, die Gerinnungsreaktion wurde durch Zugabe von 50*µ*l 0,025M CaCl₂ Lösung, welches bei 37°C temperiert wurde, gestartet. Die Gerinnungszeiten wurden in einem Kugelkoagulometer KC4A Micro bestimmt und lagen zwischen 178s und 34s.

Bei Einsatz von PEG 20kD-Faktor Xa anstelle des unmodifizierten Faktor Xa in vergleichbaren Konzentrationen im Gerinnungsassay konnte keine Gerinnung ausgelöst werden, alle gemessenen Gerinnungszeiten lagen bei >250s.

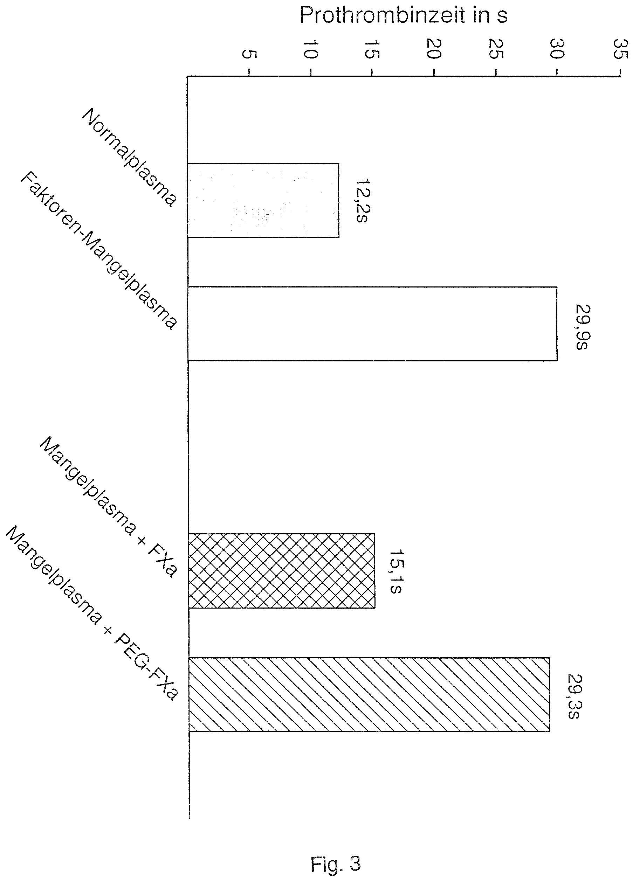

Zum Nachweis, dass PEG 20kD-Faktor Xa im Gerinnungssystem keine Aktivität ausübt, wurden außerdem folgende Untersuchungen durchgeführt: Plasma mit Mangel an Gerinnungsfaktoren (abnormal plasma, American Diagnostica Inc.) wurde Faktor Xa bzw. PEG 20kD-Faktor Xa zugesetzt (50*µ*l Plasma + 50*µ*l 8*µ*g/ml Faktor Xa in 1,5% BSA). Durch den Ausgleich des Mangels an Faktor Xa wurde die gegenüber Normalplasma (normal plasma, American Diagnostica Inc.) im Faktoren-Mangelplasma von 12,2s auf 29,9s verlängerte Prothrombinzeit auf 15,1s verkürzt. Nach Zugabe gleicher Mengen PEG 20kD-Faktor Xa wurde die Prothrombinzeit des Mangelplasmas nicht verkürzt, der PEG 20kD-Faktor Xa zeigte keine Aktivität im plasmatischen Gerinnungssystem (Abbildung 3).

### BEISPIEL 3

### Quantitative Bestimmung des niedermolekularen synthetischen direkten Faktor Xa Inhibitors Pefabloc Xa im Plasma

Zur quantitativen Bestimmung des direkten synthetischen Faktor Xa Inhibitors Pefabloc Xa (Loxo GmbH) im Plasma werden 25*µ*l PEG 20kD-Faktor Xa (1*µ*g/ml in 0,02M Tris/HCl, 0,1 M NaCl, 1,5% PEG 8.000, pH 7.4 bei RT), 25*µ*l Plasmaprobe (Citratplasma) und 100*µ*l Reaktionspuffer (0,05M Tris/HCl, 0,3M NaCl, pH 8,4 bei RT) 1min bei 37°C im Messgerät (TC4+, TECO GmbH) inkubiert. Nach Zugabe von 50*µ*l chromogenem Substrat wird die Zunahme der optischen Dichte bei 405nm aufgezeichnet (Spaltung des chromogenen Substrates und Freisetzung von p-Nitroanilin durch den nicht inhibierten Anteil des PEG 20kD-Faktor Xa).

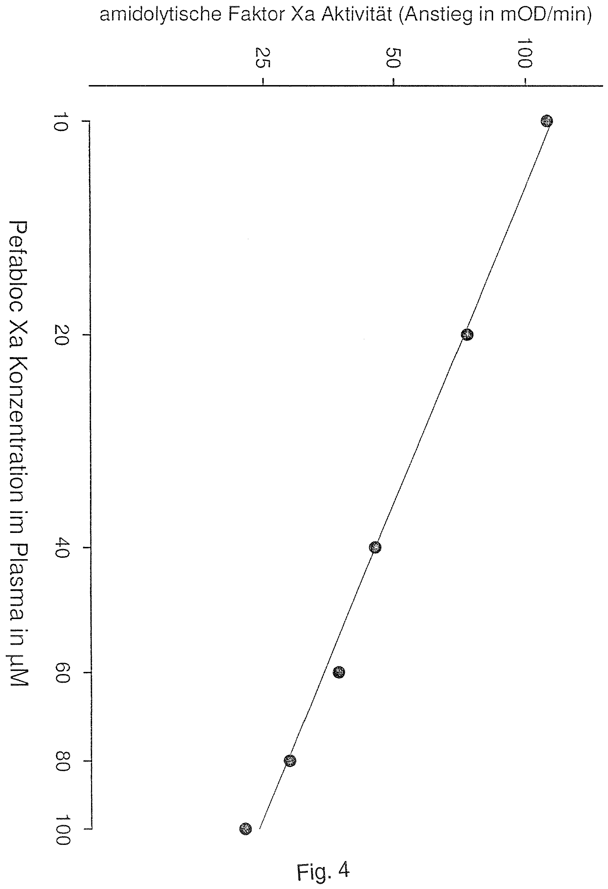

Die Aktivität des PEG 20kD-Faktor Xa wird über den Anstieg der Reaktionskurven (Änderung der optischen Dichte in mOD/min) bestimmt. Die Aktivität nimmt proportional zur Inhibitorkonzentration im Plasma ab. Es wird eine Kalibrierkurve aufgenommen, indem die PEG 20kD-Faktor Xa Aktivität bei definierten Inhibitorkonzentrationen (10*µ*M-100*µ*M) in Citratpoolplasma bestimmt wird (Abbildung 4). Mit Hilfe dieser Kalibrierkurve kann die Inhibitorkonzentration einer unbekannten Plasmaprobe über deren Aktivität ermittelt werden.

### BEISPIEL 4

### Herstellung eines PEG-Faktor IIa Konjugates

5mg boviner Faktor IIa (Thrombin, EC 3.4.21.5, Kordia.) werden zusammen mit 100mg Methoxy-Polyethylenglycol (PEG)-5.000 p-Nitrophenylcarbonat in 500*µ*l 0,05M Phosphatpuffer, pH 8,0 gelöst. Der Ansatz wurde insgesamt 2h im Kühlschrank (+2°C -+8°C) geschüttelt. Nach 30min und 60min wurden zum Ansatz weitere 45mg und nach 90min weitere 6mg Methoxy-Polyethylenglycol (PEG)-5.000 p-Nitrophenylcarbonat zugesetzt.

Das PEG 5kD-Thrombin Konjugat wurde durch Größenausschlusschromatographie an einer Hi Load Superdex 200pg 16/60 Säule mit 0,1 M NaCl bei einer Flussrate von 1 ml/min eluiert. Das PEG-Thrombin Konjugat wird als symmetrischer Peak bei einem Elutionsvolumen von 55ml zuerst eluiert (Detektion der UV Absorption bei 220nm und 280nm). Der Polymerüberschuss und die Reaktionsprodukte werden später eluiert und wurden auf diese Weise vom konjugierten Protein getrennt.

Die Protein-haltigen Fraktionen wurden in Fraktionssammlerröhrchen gesammelt, in denen PEG 8000 als Stabilisator vorgelegt wurde. Die Fraktionen mit dem höchsten Gehalt an Thrombin (gemessen über die Spaltung eines chromogenen Thrombin-Substrates) wurden gepoolt und in Aliquots bei -20°C aufgewahrt.

### BEISPIEL 5

### Quantitative Bestimmung des niedermolekularen synthetischen direkten Faktor IIa Inhibitors. Argatroban im Plasma

Zur quantitativen Bestimmung des direkten synthetischen Faktor IIa Inhibitors Argatroban (Mitsubishi Pharma) im Plasma werden 15*µ*l Plasmaprobe (Citratplasma) zu 50*µ*l chromogenem Substrat (H-D-Chg-Ala-Arg-pNA, JenAffin GmbH, 3mM in 0,05M Tris/HCl, 0,1M NaCl, pH 8,0 bei 37°C) gegeben. Die chromogene Reaktion wird durch Zugabe von 100*µ*l) PEG 5kD-Thrombin gestartet. Im Messgerät (TC4+, TECO GmbH) wird die Zunahme der optischen Dichte bei 405nm aufgezeichnet (Spaltung des chromogenen Substrates und Freisetzung von p-Nitroanilin durch den nicht inhibierten Anteil des PEG 5kD-Thrombin).

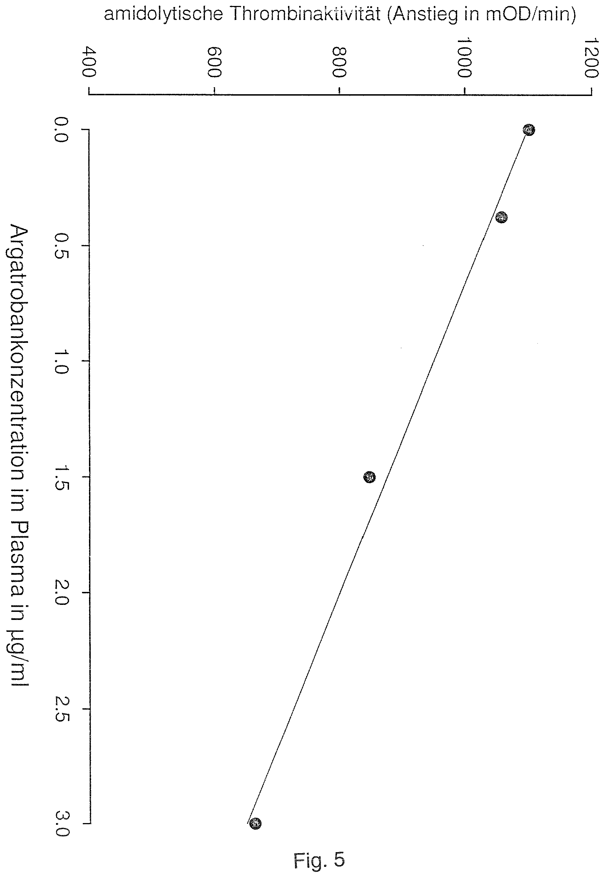

Die Aktivität des PEG 5kD-Thrombins wird über den Anstieg der Reaktionskurven (Änderung der optischen Dichte in mOD/min) bestimmt. Die Aktivität nimmt proportional zur Inhibitorkonzentration im Plasma ab. Es wird eine Kalibrierkurve aufgenommen, indem die Aktivität des PEG 5kD-Thrombin bei definierten Inhibitorkonzentrationen (0,375*µ*g/ml - 3*µ*g/ml) in Citratpoolplasma bestimmt wird (Abbildung 5). Mit Hilfe dieser Kalibrierkurve kann die Inhibitorkonzentration einer unbekannten Plasmaprobe über deren Aktivität ermittelt werden.

### BEISPIEL 6

### Einfluss von Heparin auf die amidolytische Aktivität von PEG 5kD-Thrombin

Der Einfluss von Heparin (Sigma, H-3393) in Plasma auf die amidolytische Aktivität von PEG 5kD-Thrombin wurde verglichen mit dem Einfluss von Heparin auf die amidolytische Aktivität von nicht modifiziertem Thrombin. Es wurde der chromogene Assay zur Bestimmung von direkten Thrombininhibitoren (Beispiel 5) eingesetzt: 15*µ*l Plasmaprobe (Citratplasma) werden zu 50*µ*l chromogenem Substrat (H-D-Chg-Ala-Arg-pNA, JenAffin GmbH, 3mM in 0,05M Tris/HCl, 0,1 M NaCl, pH 8,0 bei 37°C) gegeben. Die chromogene Reaktion wird durch Zugabe von 100*µ*l PEG 5kD-Thrombin bzw. Thrombin gestartet. Im Messgerät (TC4+, TECO GmbH) wird die Zunahme der optischen Dichte bei 405nm aufgezeichnet (Spaltung des chromogenen Substrates und Freisetzung von p-Nitroanilin).

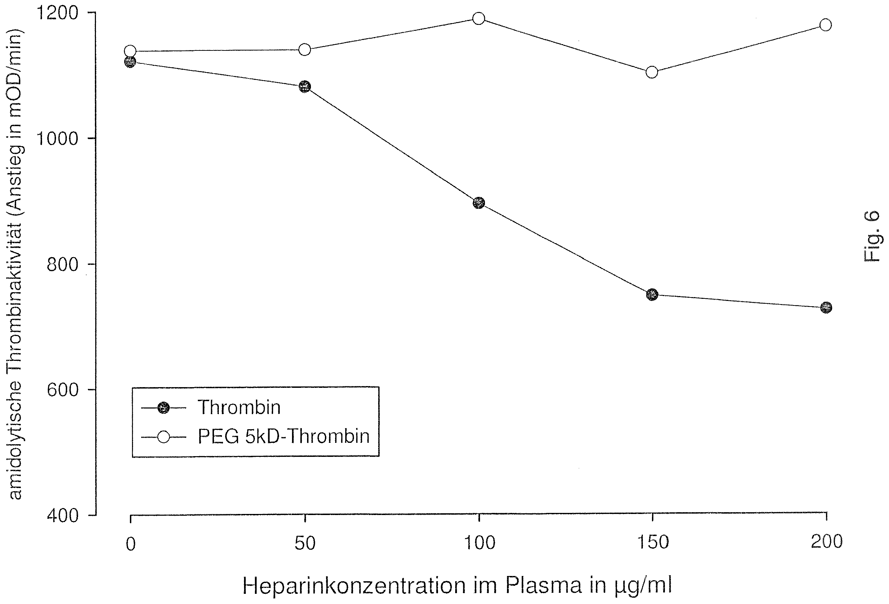

Es wurde gezeigt, dass steigende Heparinkonzentrationen in der Plasmaprobe (Heparin in Verbindung mit Plasma-Antithrombin) die Aktivität von nicht modifiziertem Thrombin hemmend beeinflussen. Auf die amidolytische Aktivität des PEG 5kD-Thrombins hatte der Heparingehalt der Plasmaprobe keinen Einfluss (Abbildung 6).
Abbildung 1: Einfluss der Antithrombin III-Inkubation auf die Aktivität von PEG 20kD-Faktor Xa sowie nicht modifiziertem Faktor Xa
Abbildung 2: Einfluss der Antithrombin III / Heparin (Low molecular weight) -Inkubation auf die Aktivität von PEG 20kD-Faktor Xa sowie nicht modifiziertem Faktor Xa
Abbildung 3: Einfluss der Zugabe von PEG 20kD-Faktor Xa sowie nicht modifiziertem Faktor Xa auf die Prothrombinzeit von Faktoren-Mangelplasma
Abbildung 4: Kalibrierkurve zur quantitativen Bestimmung des direkten Faktor Xa Inhibitors Pefabloc Xa in Plasma mittels chromogenem PEG-Faktor Xa-Assay
Abbildung 5: Kalibrierkurve zur quantitativen Bestimmung des direkten Thrombininhibitors Argatroban in Plasma mittels chromogenem PEG-Thrombin-Assay
Abbildung 6: Einfluss der Plasma-Heparinkonzentration auf die amidolytische Aktivität von PEG 5kD-Thrombin sowie nicht modifiziertem Thrombin

## Patentansprüche

1. Verfahren zur Detektion oder quantitativen Bestimmung eines Inhibitors einer Peptidase des Hämostasesystems in einer Probe, wobei die Peptidase des Hämostasesystems ein Gerinnungsfaktor ist, ausgewählt aus der Gruppe bestehend aus Faktor IIa (Thrombin), VIIa, IXa, Xa, XIa sowie Fragmenten und Mutanten der genannten Peptidasen, die mindestens 80% der Fähigkeit der genannten Peptidasen ihre Substrate des Gerinnungssystems enzymatisch umzusetzen zeigen und die Probe ausgewählt ist aus Blut, Plasma, Serum, Liquor, Urin, Schweiß oder einer anderen Flüssigkeit, umfassend
das in Kontakt bringen des Inhibitors in der Probe mit der an ein Polymer gekoppelten Peptidase des Hämostasesystems,
und das Polymer ein Polyalkylenglycol oder ein Copolymer ist, das Alkylenglycol-Einheiten umfasst, und wobei die Peptidase des Hämostasesystems durch die Polymerkopplung ihre Reaktionsfähigkeit im Gerinnungssystem verliert, aber gleichzeitig noch Inhibitoren des Gerinnungssystems mit einer molekularen Masse zwischen 100 und 2.500 Da neutralisieren und Substrate des Gerinnungssystems mit einer molekularen Masse zwischen 100 und 2.500 Da enzymatisch umsetzen kann, und entweder
(i) das Messen der Aktivität der polymergekoppelten Peptidase nach deren in Kontakt bringen mit dem Inhibitor in der Probe, und den Vergleich der Aktivität der gekoppelten Peptidase nach dem in Kontakt bringen mit der Probe mit einem oder mehreren Referenzwerten, um eine eventuelle Hemmung der Aktivität festzustellen; oder
(ii) das Messen der Gerinnungszeit der Probe nach dem in Kontakt bringen mit der gekoppelten Peptidase, und den Vergleich der Gerinnungszeit mit einem oder mehreren Referenzwerten, um eine eventuelle Änderung der Gerinnungszeit festzustellen.

2. Verfahren nach Anspruch 1, wobei es sich bei dem Inhibitor um einen direkt wirkenden Inhibitor handelt.

3. Verfahren nach den Ansprüchen 1 oder 2, wobei es sich bei dem Inhibitor um einen Wirkstoff der Klassen der direkten Faktor Xa-Inhibitoren oder der direkten Thrombininhibitoren handelt.

4. In-vitro Verfahren zur Neutralisation der inhibitorischen Aktivität eines Inhibitors einer Peptidase des Hämostasesystems mit einer molekularen Masse zwischen 100 und 2.500 Da, wobei die Peptidase ausgewählt ist aus der Gruppe bestehend aus Faktor IIa (Thrombin), VIIa, IXa, Xa, XIa sowie Fragmenten und Mutanten der genannten Peptidasen, die mindestens 80% der Fähigkeit der genannten Peptidasen zeigen, ihre Substrate des Gerinnungssystems enzymatisch umzusetzen, umfassend
das in Kontakt bringen einer Probe ausgewählt aus Blut, Plasma, Serum, Liquor, Urin, Schweiß oder einer anderen Flüssigkeit, in der der Inhibitor enthalten ist, mit einer polymergekoppelten Peptidase des Hämostasesystems,
und es sich bei dem Polymer um Polyalkylenglycol oder ein Copolymer handelt, das Alkylenglycol-Einheiten umfasst, und wobei die Peptidase durch die Polymerkopplung ihre Reaktionsfähigkeit im Gerinnungssystem verliert, aber gleichzeitig noch Inhibitoren des Gerinnungssystems mit einer molekularen Masse zwischen 100 und 2.500 Da neutralisieren und Substrate des Gerinnungssystems mit einer molekularen Masse zwischen 100 und 2.500 Da enzymatisch umsetzen kann,
und das Binden des Inhibitors durch Reaktion mit der polymergekoppelten Peptidase.

5. Verfahren nach Anspruch 4, wobei die Probe aus dem Blut eines Patienten gewonnen wird, der mit einem Arzneimittel behandelt wird bzw. wurde, das einen Inhibitor einer Peptidase des Hämostasesystems als Wirkstoff enthält und wobei nach dem in Kontakt bringen der Probe mit der gekoppelten Peptidase der mit dem Arzneimittel verabreichte Inhibitor durch die Peptidase gebunden und damit neutralisiert wird.

6. Verfahren nach Anspruch 4 oder 5, wobei zusätzlich nach der Neutralisation des Inhibitors Tests zur Diagnose von Störungen im Hämostasesystem oder zur Aktivität von Substanzen, die in das Hämostasesystem eingreifen, an der Probe durchgeführt werden.

## Claims

1. A process for the detection or quantitative determination of an inhibitor of a peptidase of the hemostatic system in a sample, wherein said peptidase of the hemostatic system is a coagulation factor selected from the group consisting of factors IIa (thrombin), VIIa, IXa, Xa, XIa, and fragments and mutants of the mentioned peptidases showing at least 80% of the capability of the mentioned peptidases of converting their substrates of the coagulation system enzymatically, and said sample is selected from blood, plasma, serum, cerebrospinal fluid, urine, sweat or another fluid, comprising
contacting the inhibitor in the sample with said peptidase of the hemostatic system coupled to a polymer;
wherein said polymer is a polyalkylene glycol or a copolymer including alkylene glycol units, and wherein said peptidase of the hemostatic system loses its reactivity in the coagulation system because of the polymer coupling, but is still able to neutralize inhibitors of the coagulation system with a molecular weight of from 100 to 2,500 Da and to convert substrates of the coagulation system with a molecular weight of from 100 to 2,500 Da enzymatically, and either
(i) measuring the activity of the polymer-coupled peptidase after having been contacted with the inhibitor in the sample, and comparing the activity of the coupled peptidase after said contacting with the sample with one or more reference values in order to determine an inhibition of the activity, if any; or
(ii) measuring the clotting time of the sample after having been contacted with the coupled peptidase, and comparing the clotting time with one or more reference values in order to determine a change of the clotting time, if any.

2. The process according to claim 1, wherein said inhibitor is a directly acting inhibitor.

3. The process according to claims 1 or 2, wherein said inhibitor is an active substance from the classes of direct factor Xa inhibitors or direct thrombin inhibitors.

4. An in-vitro method of neutralizing the inhibitory activity of an inhibitor of a peptidase of the hemostatic system with a molecular weight of from 100 to 2,500 Da, wherein said peptidase is selected from the group consisting of factors IIa (thrombin), VIIa, IXa, Xa, XIa, and fragments and mutants of the mentioned peptidases showing at least 80% of the capability of the mentioned peptidases of converting their substrates of the coagulation system enzymatically, comprising
contacting a sample selected from blood, plasma, serum, cerebrospinal fluid, urine, sweat or another fluid, in which the inhibitor is contained, with a polymer-coupled peptidase of the hemostatic system,
wherein said polymer is a polyalkylene glycol or a copolymer including alkylene glycol units, and wherein said peptidase loses its reactivity in the coagulation system because of the polymer coupling, but is still able to neutralize inhibitors of the coagulation system with a molecular weight of from 100 to 2,500 Da and to convert substrates of the coagulation system with a molecular weight of from 100 to 2,500 Da enzymatically, and
binding the inhibitor by a reaction with said polymer-coupled peptidase.

5. The process according to claim 4, wherein said sample is obtained from the blood of a patient who is being or was treated with a medicament containing an inhibitor of a peptidase of the hemostatic system as an active ingredient, and wherein the inhibitor administered with the medicament is bound and thereby neutralized by the peptidase after the sample has been contacted with said coupled peptidase.

6. The process according to claim 4 or 5, wherein tests for the diagnosis of disorders in the hemostatic system or of the activity of substances interfering with the hemostatic system are additionally performed with the sample after said neutralization of the inhibitor.

## Revendications

1. Procédé destiné à la détection ou à la détermination quantitative d'un inhibiteur d'une peptidase du système de l'hémostase dans un échantillon, où la peptidase du système de l'hémostase est un facteur de coagulation sélectionné dans le groupe constitué du facteur IIa (thrombine), VIIa, IXa, Xa, XIa, ainsi que des fragments et des mutants des peptidases mentionnées, lesquelles peptidases montrent au moins 80% de la capacité des peptidases mentionnées à transformer de manière enzymatique leurs substrats du système de coagulation et où l'échantillon est sélectionné à partir du sang, du plasma, du sérum, de la liqueur, de l'urine, de la sueur ou d'un autre liquide, comprenant
la mise en contact de l'inhibiteur dans l'échantillon avec la peptidase du système de l'hémostase, laquelle peptidase est couplée par un polymère,
et le polymère est un polyalkylène glycol ou un copolymère qui comprend des motifs alkylène glycol et où la peptidase du système de l'hémostase perd sa capacité de réaction dans le système de coagulation par le couplage avec le polymère, tout en pouvant encore neutraliser dans le même temps des inhibiteurs du système de coagulation dont la masse moléculaire est comprise entre 100 et 2.500 Da et en pouvant transformer de manière enzymatique des substrats du système de coagulation dont la masse moléculaire est comprise entre 100 et 2.500 Da, ainsi que
(i) soit la mesure de l'activité de la peptidase couplée au polymère à l'issue de sa mise en contact avec l'inhibiteur dans l'échantillon ainsi que la comparaison de l'activité de la peptidase couplée au polymère à l'issue de la mise en contact de l'échantillon avec une ou plusieurs valeurs de référence afin de constater une inhibition éventuelle de l'activité ;
(ii) soit la mesure du temps de coagulation de l'échantillon à l'issue de la mise en contact avec la peptidase couplée au polymère, ainsi que la comparaison du temps de coagulation avec une ou plusieurs valeurs de référence afin de constater un changement éventuel du temps de coagulation.

2. Procédé selon la revendication 1, dans lequel l'inhibiteur en question est un inhibiteur agissant directement.

3. Procédé selon les revendications 1 ou 2, dans lequel l'inhibiteur en question est une substance active des classes des inhibiteurs directs du facteur Xa ou des inhibiteurs directs de thrombine.

4. Procédé in vitro destiné à la neutralisation de l'activité inhibitrice d'un inhibiteur d'une peptidase du système de l'hémostase dont la masse moléculaire est comprise entre 100 et 2.500 Da, où la peptidase est sélectionnée dans le groupe constitué du facteur IIa (thrombine), VIIa, IXa, Xa, XIa ainsi que des fragments et des mutants des peptidases mentionnées, lesquelles peptidases montrent au moins 80% de la capacité des peptidases mentionnées à transformer de manière enzymatique leurs substrats du système de coagulation, comprenant
la mise en contact d'un échantillon, sélectionné à partir de sang, de plasma, de sérum, de liqueur, d'urine, de sueur ou d'un autre liquide, au sein duquel l'inhibiteur est contenu, avec une peptidase du système de l'hémostase couplée à un polymère,
et le polymère en question est un polyalkylène glycol ou un copolymère qui comprend des motifs alkylène glycol, et où la peptidase perd sa capacité de réaction dans le système de coagulation par le couplage avec le polymère, tout en pouvant encore neutraliser dans le même temps des inhibiteurs du système de coagulation dont la masse moléculaire est comprise entre 100 et 2.500 Da et en pouvant transformer de manière enzymatique des substrats du système de coagulation dont la masse moléculaire est comprise entre 100 et 2.500 Da,
et la liaison de l'inhibiteur par la réaction avec la peptidase couplée par polymère.

5. Procédé selon la revendication 4, dans lequel l'échantillon est obtenu à partir du sang d'un patient qui est ou a été traité avec un produit médicinal, lequel contient comme substance active un inhibiteur d'une peptidase du système de l'hémostase et où l'inhibiteur administré par l'intermédiaire du produit médicinal est lié à la peptidase à l'issue de la mise en contact de l'échantillon avec la peptidase couplée et est par conséquent neutralisé.

6. Procédé selon la revendication 4 ou 5, où, à l'issue de la neutralisation de l'inhibiteur, des tests destinés au diagnostic des troubles du système de l'hémostase ou destinés à l'activité de substances qui interviennent dans le système de l'hémostase, sont réalisés sur l'échantillon.
